(19) Europäisches Patentamt
European Patent Office
Office européen des brevets

(11) **EP 2 369 340 B1**

(12) **EUROPEAN PATENT SPECIFICATION**

(45) Date of publication and mention
of the grant of the patent:
**25.09.2013 Bulletin 2013/39**

(51) Int Cl.:
**G01N 33/46** *(2006.01)*  **G01N 29/07** *(2006.01)*

(21) Application number: **11159150.9**

(22) Date of filing: **22.03.2011**

(54) **Evaluating the elasticity of wooden elements at a reference temperature**

Bestimmung der Elastizität von Holzelementen bei einer Referenztemperatur

Détermination d'élasticité d'éléments en bois à température de référence

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.03.2010 IT VR20100052**

(43) Date of publication of application:
**28.09.2011 Bulletin 2011/39**

(73) Proprietor: **MICROTEC S.r.l.**
**39042 Bressanone (Bolzano) (IT)**

(72) Inventor: **Bacher, Martin**
**39030, Pfalzen (IT)**

(74) Representative: **Ponchiroli, Simone**
**Ruffini Ponchiroli e Associati S.r.l.**
**Via Caprera, 6**
**37126 Verona (IT)**

(56) References cited:
**US-A1- 2008 197 054**

• **HELMUT BÄCHLE ET AL: "The influence of temperature on the velocity of sound in green pine wood", HOLZ ALS ROHUND WERKSTOFF ; EUROPEAN JOURNAL OF WOOD AND WOOD PRODUCTS, SPRINGER, BERLIN, DE LNKD- DOI: 10.1007/S00107-005-0083-7, vol. 64, no. 5, 8 March 2006 (2006-03-08), pages 429-430, XP019434510, ISSN: 1436-736X**
• **SANDOZ J. L.: "Moisture content and temperature effect on ultrasound timber grading", WOOD SCI. TECHNOL., vol. 27, no. 5, 1993, pages 373-380, XP002604787, ISSN: 0043-7719, DOI: 10.1007/BF00192223**
• **SANDOZ J. L.: "Grading of construction timber by ultrasound", WOOD SCI. TECHNOL., vol. 23, no. 1, 1989, pages 95-108, XP002604788, ISSN: 0043-7719, DOI: 10.1007/BF00350611**
• **ROSS R J ET AL: "STRESS WAVE NONDESTRUCTIVE EVALUATION OF LOGS TO PREDICT STRUCTURAL PRODUCT QUALITY", MATERIALS SCIENCE FORUM, AEDERMANNSFDORF, CH, vol. 210-213, 1 January 1996 (1996-01-01), pages 109-114, XP009048794, ISSN: 0255-5476**

**Description**

This invention relates to a method for evaluating the elasticity of wooden elements at a reference temperature. In particular, the invention is intended for application to logs, planks and to wooden elements having an elongate shape in general. Therefore, hereinafter explicit reference is often made to that type of wooden elements.

At present there are many prior art non-destructive methods for evaluating the elasticity of logs and planks (there are also destructive methods, although they are not of interest for the purposes of this invention). Moreover, amongst all of the prior art non-destructive methods, this invention relates to the method which comprises evaluation of the elasticity based on the density of the wooden element and on its natural frequency (or resonant frequency). The modulus of elasticity E of a wooden element is defined by the equation:

$$E = V^2 \rho$$

where V is the velocity of propagation of sound in the wooden element and p is the density of the wooden element.

Since the velocity of propagation is linked to the natural frequency f of the log or the plank, from the equation:

$$V = 2 L f$$

where L is the length of the log or of the plank, the modulus of elasticity E may also be expressed as:

$$E = 4 L^2 f^2 \rho$$

An example of patents which describe a method for evaluating the elasticity based on density and natural frequency/velocity of propagation is patents DE 44 35 975 and US 6,026,089.

In accordance with that method a sound wave is generated in the wooden element by striking it with a "hammer" at one end in such a way as to make it vibrate. A detection device (such as a microphone or a laser sensor) located at the same end or at the opposite end then detects the resonant frequency of the wooden element. A processing unit then immediately determines the velocity of propagation of the sound wave in the wooden element.

However, this prior art technology has several disadvantages linked to the ways in which it is implemented in practice.

Indeed, depending on the geographical location of the wood processing plants, the season, and the type of works (open air or indoors), the environmental conditions in which work is carried out may vary significantly, in particular with reference to the temperature.

Studies carried out have shown how the temperature of the wood has a more or less significant influence on the velocity of propagation of a sound wave inside it (therefore, on the resonant frequency) and, therefore, on its modulus of elasticity. In particular, such studies have shown how such variations are particularly significant when the wood is frozen. In that situation, the water contained in the wood transforms into ice and affects the mechanical behaviour of the log much more than when it is in the liquid state.

Amongst the various studies published, reference may be made to articles by Helmut Bächle et al, "The influence of temperature on the velocity of sound in green pine wood", Holz als Roh- und Werkstoff (2006) 64: 429-430 and Sandoz, "Moisture content and temperature effect on ultrasound timber grading", Wood Sci. Technol 27: 373-380 (1993), as well as the degree thesis by Julian Moreno Chan, "Moisture content in radiata pine wood: Implications for wood quality and water-stress response", School of Forestry, College of Engineering, University of Canterbury 2007.

Consequently, it seems clear how conventional methods, described in the above-mentioned patents, are unable to provide reliable results whatever the temperature of the log.

Moreover, wood, as such, is a material which has a high thermal inertia, so that it is practically impossible even to only suppose that the log can be brought to the correct temperature before measuring its natural frequency. In fact, to be able to guarantee satisfactory productivity, entire sheds at a controlled temperature would be required, in which to store the logs for several days before measuring the natural frequency at the correct temperature, even more so in countries where in winter temperatures may drop well below 0°C. To attempt to overcome that disadvantage, patent application US 2008/0197054 comprises correcting the values measured based on the temperature of the log, in such a way as to estimate the corresponding values at a reference temperature (for example at 20°C). Therefore, in accordance with that document, in addition to the velocity of the sound wave, the surface temperature of the log is also measured at one of its end faces.

However, even that solution is unable to always achieve the result hoped for.

Considering that wood has a high thermal inertia which causes a related slowness in its temperature variations, the correction based on the surface temperature may be considered ample when it is reasonable to consider the external temperature not very different from the internal temperature (in each case the technician in the sector will not have difficulty evaluating this matter in quantitative terms). Instead, to better understand this aspect it is important to consider that normally the various wooden elements (and in particular logs) before being examined and then cut, remain piled up outdoors for relatively lengthy periods of time, therefore achieving a certain thermal equilibrium with the environment. However, while the log internal temperature varies very slowly, the surface temperature may vary more rapidly (for example due to exposure to the sun).

Therefore, when the time comes to examine and cut a log, it is picked up from the relative heap and sent along the processing line. However, when it is picked up the log is affected by the evolution of the ambient temperature in the preceding hours. If the ambient temperature remained practically constant for a long time, the outer surface temperature may be considered close to the log internal temperature. However, that is very unlikely.

In most cases it is likely that during the time before the log was picked up, it was exposed to significant variations in the external temperature (for example due to the rays of the sun), which modified the surface temperature but which, due to the thermal inertia of the log, did not affect the log internal temperature.

Further, if the processing line is located inside works, the internal temperature may be considerably different to the external temperature (in particular during winter in regions with a harsh climate) . Consequently, when the log is brought into the works, its temperature begins to vary from the surface.

Again in this case, given the high thermal inertia of wood, if the time which elapses between entering the works and measuring the temperature and actual velocity of propagation of the sound wave is limited, the variation in the surface temperature will be minimal and may be assumed to be negligible for the purposes of this invention (in that case only the previous variations in the external temperature will count). In contrast, when said time is longer, the surface temperature may again no longer sufficiently reflect the log internal temperature, and consequently a correction based on the temperature measured in this way is meaningless.

In this situation the technical purpose which forms the basis of this invention is to provide a method for evaluating the modulus of elasticity of wooden elements, such as logs and planks, which overcomes the above-mentioned disadvantages.

In particular, the technical purpose of this invention is to provide a method for evaluating the elasticity of wooden elements, such as logs and planks, which allows the elasticity at a reference temperature to be determined with a good degree of precision, irrespective of the temperature of the log or the temperature to which the log was exposed in the preceding hours.

This invention also has for a technical purpose to provide a method for evaluating the elasticity of wooden elements, such as logs and planks, which does not require lengthy operations for conditioning the temperature of the logs before evaluating the elasticity. The technical purpose specified and the aims indicated are substantially achieved by a method for evaluating the elasticity of wooden elements, such as logs and planks, as described in the appended claims.

Further features and the advantages of this invention are more apparent from the detailed description of several preferred, non-limiting embodiments of a method for evaluating the elasticity of wooden elements, such as logs and planks, illustrated with reference to the accompanying drawings, in which:

- Figure 1 is a perspective view of a line for measuring the natural frequency of wooden elements (logs);
- Figure 2 is a schematic view of several operating steps of an embodiment of the method according to this invention;
- Figure 3 is a schematic graph of the trend of the velocity of propagation of a sound wave inside a wooden element, depending on the temperature of the wooden element;
- Figure 4 illustrates a further operating step of the method according to this invention;
- Figure 5 illustrates the operating step of Figure 4 as it proceeds;
- Figure 6 shows a step of loading a log on a possible device for determining the velocity of propagation of the sound in it;
- Figure 7 shows the device of Figure 6 with the log in the operating position for determining the velocity of propagation of the sound in it; and
- Figure 8 shows a step of unloading the log from the device of Figure 6. In accordance with this invention, the method for evaluating the elasticity of wooden elements 1 such as logs and planks comprises in general evaluation of the elasticity of a wooden element 1 based on the velocity of propagation of a sound wave inside it (or, equivalently, based on its natural frequency).

To do this, the method comprises first an operating step of generating a sound wave of the wooden element 1 being examined.

In the preferred embodiment, the sound wave is generated by striking the wooden element 1 with a suitable "hammer" 2 operated by means of an actuator 3 in such a way as to strike the wooden element 1 once.

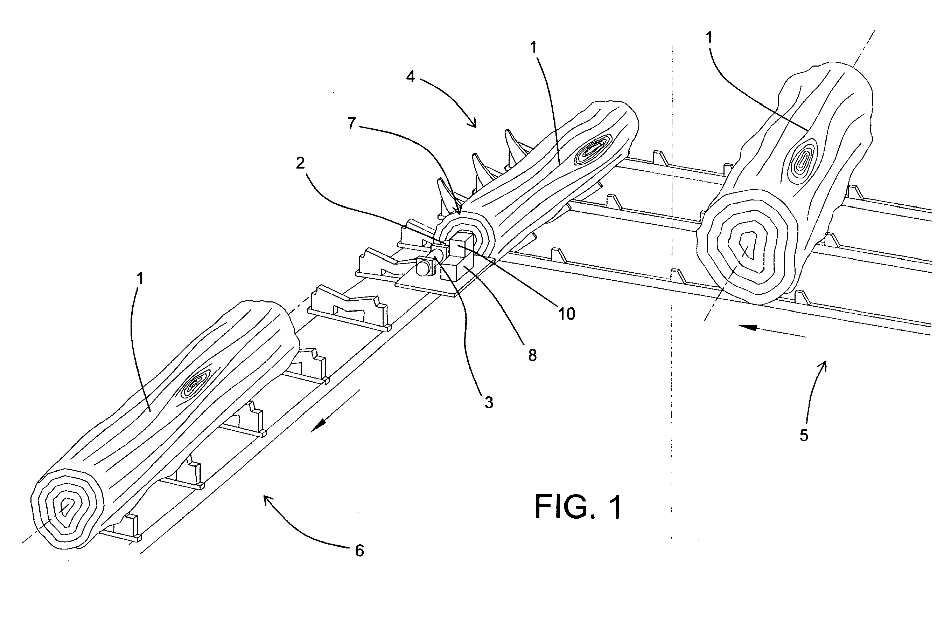

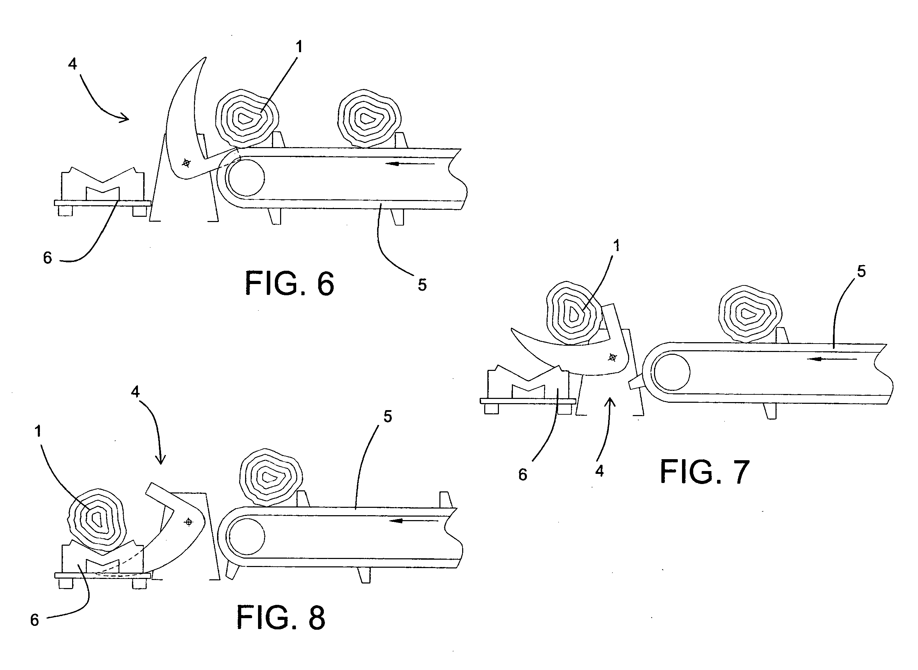

In the embodiment illustrated in Figures 1, 2 and 6 to 8 (corresponding to that which is the subject matter of US 2010/0064810), said step is performed at a device 4 for transferring the wooden elements 1 from a first feed line 5 to a second feed line 6 which is transversal to the first feed line 5. When the wooden element 1 (in the case illustrated a log) is supported by the transfer device 4 as illustrate in Figures 1 and 7, its end 7 is at the "hammer" 2.

The second step of the method according to this invention is that of at least indirectly measuring the actual velocity of propagation of the sound wave in the wooden element 1. It should be noticed that the expression "actual velocity of propagation" refers to the velocity with which the sound wave effectively propagates in the wooden element 1 in the conditions in which the latter is. It should also be noticed that in this text, the expression "at least indirect measuring/determining the velocity of propagation" will always mean measuring/determining either the velocity itself or another physical quantity linked to it by a formula which is known, given the wooden element (for example proportional, such as the resonant frequency or the multiple frequencies of the resonant frequency - the higher harmonics).

In the embodiment preferred and illustrated, this is achieved by means of a detector 8, located close to the free end 7 of the wooden element 1 which is struck, and connected to a processing unit 9. The detector 8, which may be either a sound microphone or a vibration laser detector 8, therefore detects the log resonant frequency in the known way. At that point, the length of the log being known (which may be measured at any time before or after), the processing unit 9 can at least indirectly determine the actual velocity of propagation of the sound wave inside the wooden element 1.

There is a further operating step of measuring the temperature of the wooden element 1, and a step of using that information for evaluating the elasticity according to the ways described in more detail below.

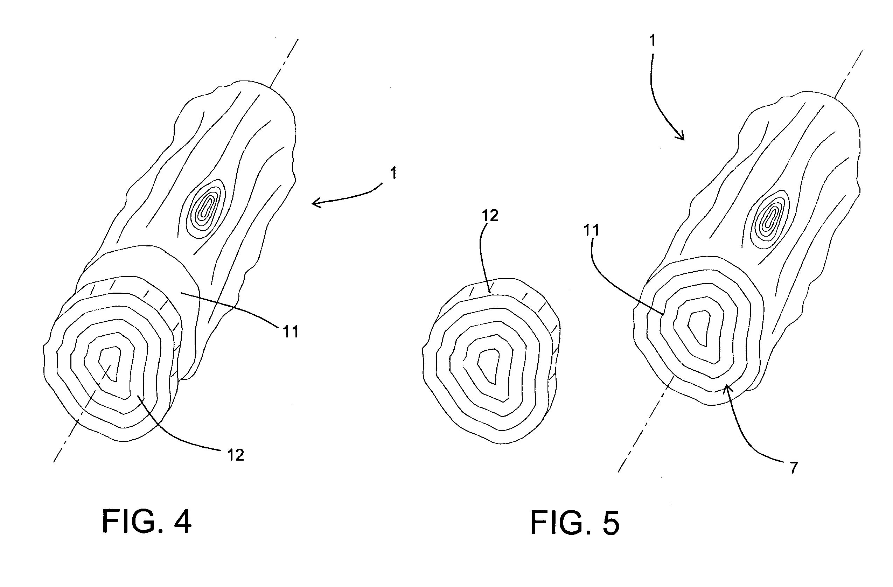

In accordance with this invention, the method, before the step of measuring the temperature, comprises a step of removing part of the wooden element 1 in such a way as to make a new external face 11 on it which is substantially at the same temperature as the inside of the wooden element 1. As illustrated in Figures 5 and 6, in the case of logs, planks and other elongate wooden elements 1, the removal step preferably comprises the removal of an end portion 12 of the wooden element.

Thus, the step of measuring the temperature is carried out by measuring the surface temperature at the new external face 11. Moreover, advantageously, the removal step and the measurement step are carried out in sequence within a time which is such that the surface temperature of the new external face 11 is not in the meantime subject to variations that are significant for application of the method (according to the criteria indicated above).

Once the temperature of the wood and the actual velocity of propagation of the sound wave have been measured, the method according to this invention may comprise at least two different ways of proceeding for evaluating the elasticity at the reference temperature, that is to say:

- using the temperature value measured and the actual velocity of propagation measured to calculate a reference velocity of propagation at the reference temperature, then evaluating the elasticity at the reference temperature using the reference velocity of propagation; or
- evaluating the elasticity at the temperature measured using the actual velocity of propagation, then using the elasticity evaluated in that way and the temperature value measured, for evaluating the elasticity at the reference temperature. These two ways of proceeding are now described in detail one after the other.

In accordance with the first way of proceeding indicated above, the method comprises a step of determining a reference velocity of propagation of the sound wave or of the other quantity linked to it, which will then be used for evaluating the elasticity.

The method according to the invention comprises various possible embodiments for this.

Indeed, a first embodiment comprises a comparison of the temperature measured and a preset threshold value (advantageously set, in the preferred embodiments, as equal to 0°C). In this case, if the temperature measured is greater than the preset threshold value, the reference velocity of propagation may be assumed to be equal to the actual velocity of propagation measured. If, in contrast, the value measured is less than the preset threshold value, there is an operating step of calculating the reference velocity of propagation of the sound wave in the wooden element 1, as the velocity of propagation at the preset reference temperature. In other words, the value of the actual velocity of propagation measures is corrected, based on the temperature of the wood, in such a way as to obtain a value of the velocity of propagation which corresponds to that which could be obtained by measuring the velocity itself with the wood at the reference temperature. Said calculation is therefore carried out based on the measurements of the actual velocity of propagation and the temperature of the wooden element 1.

In the preferred embodiment, the calculation is carried out in practice starting with known data which must have been determined previously. In general, they are tables which, for each type of wood, provide a series of trends of the velocity of propagation depending on the temperature, parametrised according to other features such as the dimensions of the wooden elements 1, the moisture, the density, etc. (features which, if necessary, are first measured or estimated).

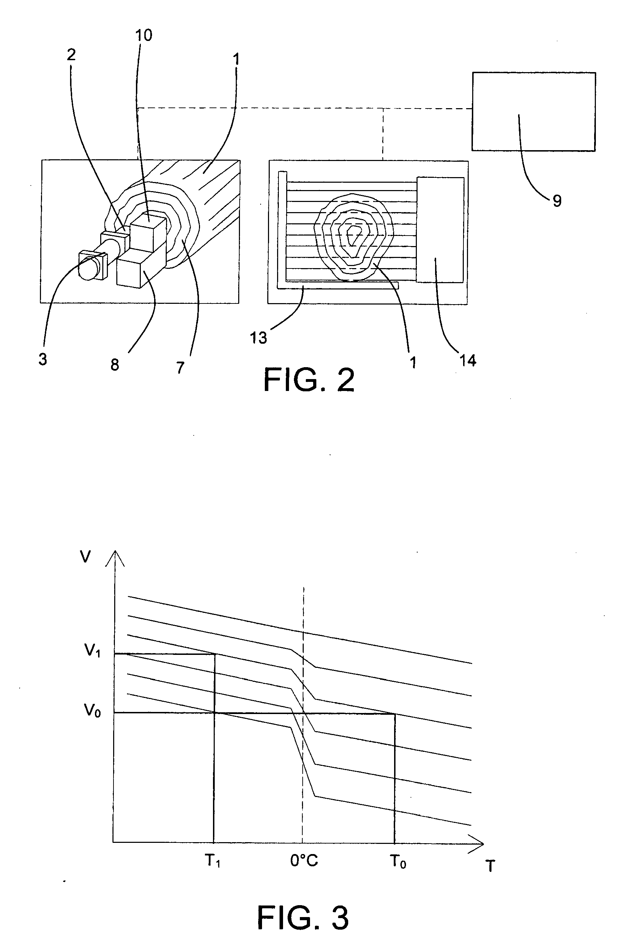

Based on said tables, simple interpolation operations can be used to establish, starting from a certain velocity $V_1$ measured at a temperature $T_1$, the set of values (characteristic trend) to which the wooden element 1 belongs and therefore to determine what is the related reference velocity of propagation $V_0$ at the reference temperature To. That method is qualitatively illustrated in Figure 3, which shows six different trends of the velocity of propagation according to the temperature of the wood, parametrised according to the moisture (similar curves are also obtained for other parameters).

Finally, advantageously, the reference temperature $T_0$ is set as equal to 20°C.

In contrast, in a second embodiment of the first way of proceeding with the method according to this invention, the step of calculating the velocity of propagation at the reference temperature is carried out for any temperature measured, without any comparison of the latter with preset threshold values. The ways of implementing the method are the same as those described above relative to the case in which the temperature measured is below a preset threshold value.

Finally, the first way of proceeding with the method according to this invention comprises a subsequent step of evaluating the elasticity of the wooden element 1 based on the velocity of propagation calculated at the reference temperature (or, if necessary, based on the other quantity linked to it) .

With reference to the second way of proceeding with the method according to this invention, after measuring the temperature and the actual velocity of propagation, as already indicated the method comprises evaluation of the elasticity at the temperature measured using in the evaluation the actual velocity of propagation, and subsequently using the elasticity evaluated in that way and the temperature value measured, to evaluate the elasticity at the reference temperature.

For this purpose the method according to the invention comprises various possible embodiments, substantially similar to those described above as regards the first way of proceeding with the method.

Indeed, a first embodiment comprises a comparison of the temperature measured and a preset threshold value (advantageously set, in the preferred embodiments, as equal to 0°C) . In this case, if the temperature measured is greater than the preset threshold value, the elasticity at the reference temperature may be assumed to be equal to that measured. If, in contrast, the temperature value measured is less than the preset threshold value, there is an operating step of evaluating the elasticity of the wooden element 1 at the reference temperature. In other words, the elasticity evaluated at the temperature measured is corrected based on the temperature, in such a way as to obtain a value which corresponds to that which could be obtained by evaluating the elasticity with the wood at the reference temperature.

In the preferred embodiment, the evaluation is carried out in practice starting with known data which must have been determined previously. In general, they are tables which, for each type of wood, provide a series of trends of the elasticity depending on the temperature, parametrised according to other features such as the dimensions of the wooden elements 1, the moisture, the density, etc. Based on said tables, simple interpolation operations can be used to establish, starting from a certain modulus of elasticity $E_1$ measured at a temperature $T_1$, the set of values (characteristic trend) to which the wooden element 1 belongs and therefore to determine what is the related modulus of elasticity $E_0$ at the reference temperature To. Said method is qualitatively similar to the one described above relative to the velocity of propagation.

Finally, advantageously, again in this case the

reference temperature $T_0$ is set as equal to 20°C.

In contrast, in a second embodiment of the second way of proceeding with the method according to this invention, the step of evaluating the modulus of elasticity at the reference temperature is carried out for any temperature measured, without any comparison of the latter with preset threshold values. The ways of implementing the method are the same as those described above for the case in which the temperature measured is below a preset threshold value.

As already indicated, it is known that the modulus of elasticity value may be calculated using the formula:

$$E = V^2 \rho$$

where V is the velocity of propagation (actual or reference) and p is the mean density of the wooden element 1.
Alternatively, since the velocity of propagation is linked to the natural frequency f of the log or the plank, from the equation:

$$V = 2 L f$$

where L is the length of the log or of the plank, the modulus of elasticity E may also be calculated as:

$$E = 4 L^2 f^2 \rho$$

In the context of this invention, the expression "evaluating the elasticity" refers to both actual calculation of the modulus of elasticity using the formulas shown above, and to simple evaluation of the elasticity excluding factors considered known. For example, if the density is assumed to be constant, the evaluation of the elasticity may be carried out considering only the factor V or only the factor V2. Depending on the embodiments, the density of the wooden element 1 may be managed in different ways. In particular, in a first rough case of application, for a predetermined type of wood it may be assumed even to be constant both in a predetermined group of wooden elements of similar origin and in general (applies in particular with reference to green logs, that is to say, which were cut a short time ago). In contrast, in more precise applications, the density of the individual wooden element may be used. In this case, the density may be a parameter already known at the moment of implementing the method according to this invention, or there may also be a specific step of determining the density. For example, Figure 2 shows the case in which the density of a log is determined, in a known way, by means of an X-ray examination of the log. In Figure 2 the log is supported by a carriage 13 and is laterally struck by a beam of rays emitted by an X-ray device 14. Alternatively, the density may also be calculated as a simple ratio of the weight and volume of the log.

Finally, it should be noticed that for correct application of the method according to this invention in accordance with the specific preferred embodiments described above, it is necessary to have available the mapping of the trend of the velocity of propagation and/or of the elasticity according to the temperature parameterised for the possible different operating parameters. Calibration consists of preliminary measuring, for different types of wood and different parameters of the wooden element 1 (such as the density and the moisture), the velocity of propagation of a sound wave and/or the elasticity for a plurality of different possible operating temperatures of the wood.

Although calibration is preferably carried out only once for all of the apparatuses intended to apply the method according to this invention, depending on requirements the method itself may comprise a preliminary calibration step.

This invention brings important advantages.

First, the method according to this invention allows evaluation with a good degree of precision of the elasticity of the wooden element at the reference temperature irrespective of the current temperature of the wooden element or its temperature during the preceding hours.

Second, said method does not require lengthy operations for conditioning the temperature of the logs before carrying out the evaluation of the modulus of elasticity, and, therefore, substantially does not affect the productivity and dimensions of the plants.

It should also be noticed that this invention is relatively easy to produce and that even the cost linked to implementing the invention is not very high.

The invention described above may be modified and adapted in several ways without thereby departing from the scope of the inventive concept.

**Claims**

1. A method for evaluating the elasticity of a wooden element such as a log or a plank at a reference temperature, comprising the operating steps of:

> generating a sound wave in the wooden element (1);
> determining the actual velocity of propagation of the sound wave in the wooden element (1) or another quantity linked to it by a known formula;
> measuring the temperature of the wooden element (1); and
> using the temperature measured and the actual velocity of propagation measured, or the other quantity linked to it, for evaluating the elasticity at a preset reference temperature;

**characterised in that** the step of measuring the temperature is carried out by measuring the internal temperature of the wooden element (1), and **in that** it also comprises, before the step of measuring the temperature, a step of removing part of the wooden element (1) in such a way as to create a new external face (11) on it, the step of measuring the temperature being carried out by measuring the surface temperature at the new external face (11).

2. The method according to claim 1, **characterised in that** the removal step and the measurement step are carried out in sequence within a time which is such that the surface temperature of the new external face (11) is not in the meantime subject to variations that are significant for application of the method.

3. The method according to claim 1 or 2, **characterised in that** the removal step comprises removing an end portion (12) of the wooden element.

4. The method according to any of the foregoing claims, **characterised in that** the step of using the temperature and the actual velocity of propagation or the other quantity linked to it, for evaluating the elasticity at the reference temperature, comprises the operating step of calculating, based on said measurements of the actual velocity of propagation and the temperature of the wooden element (1), a reference velocity of propagation of the sound wave in the wooden element (1) at the reference temperature, or another quantity linked to it by a known formula, and subsequently the operating step of evaluating the elasticity of the wooden element (1) at the reference temperature, based on the reference velocity of propagation.

5. The method according to claim 4, **characterised in that** when the temperature measured is greater than 0°C, the step of calculating the velocity of propagation, or the other quantity linked to it, at the reference temperature is carried out by assuming that it is equal to that measured.

6. The method according to any of the claims from 1 to 3, **characterised in that** the step of using the temperature and the actual velocity of propagation, or the other quantity linked to it, for evaluating the elasticity at the reference temperature, comprises the operating step of evaluating the elasticity at the temperature measured based on said measurements of the actual velocity of propagation or the other quantity linked to it, and the subsequent operating step of evaluating the elasticity of the wooden element (1) at the reference temperature based on the temperature measured and the previous evaluation carried out at the temperature measured.

**7.** The method according to claim 6, **characterised in that**, when the temperature measured is greater than 0°C, the step of evaluating the elasticity at the reference temperature is carried out by assuming that it is equal to that at the temperature measured.

**8.** The method according to any of the foregoing claims, **characterised in that** the reference temperature is set as equal to 20 °C.

**9.** The method according to any of the foregoing claims, characterised 4 in that a calculation of the modulus of elasticity is carried out based on the density of the wooden element (1) previously determined or estimated.

**10.** The method according to any of the foregoing claims, **characterised in that** it also comprises a preliminary calibration step.

**11.** The method according to claim 10, **characterised in that** the calibration step comprises measuring, for various types of wood and various parameters of the wooden element (1), the velocity of propagation of a sound wave or the other quantity linked to it, and/or evaluating the elasticity, for a plurality of different possible operating temperatures.

**Patentansprüche**

**1.** Verfahren zur Bestimmung der Elastizität eines Holzelementes, solches wie ein Stamm oder eine Planke, bei einer Referenztemperatur, enthaltend die folgenden Vorgehensphasen:

- Erzeugen einer Schallwelle in dem Holzelement (1);
- Bestimmung der aktuellen Geschwindigkeit der Fortpflanzung der Schallwelle in dem Holzelement (1) oder einer anderen, mit dieser zusammenhängenden Grösse, durch eine bekannte Formel;
- Messen der Temperatur des Holzelementes (1); und
- Verwendung der gemessenen Temperatur und der gemessenen aktuellen Geschwindigkeit oder der anderen, mit dieser zusammenhängenden Grösse, um die Elastizität bei einer vorgegebenen Referenztemperatur zu bestimmen;

**dadurch gekennzeichnet, dass** die Phase des Messens der Temperatur durch das Messen der Innentemperatur des Holzelementes (1) erfolgt, und dadurch, dass es ebenfalls vor der Phase des Messens der Temperatur eine Phase des Entfernens von einem Teil des Holzelementes (1) enthält, und zwar auf solche Weise, dass eine neue Aussenfläche (11) an diesem geschaffen wird, wobei die Phase des Messens der Temperatur durch das Messen der Oberflächentemperatur an der neuen Aussenfläche (11) durchgeführt wird.

**2.** Verfahren nach Patentanspruch 1, **dadurch gekennzeichnet, dass** die Phase des Entfernens und die Phase des Messens aufeinanderfolgend innerhalb einer Zeit durchgeführt werden, die eine solche ist, dass die Oberflächentemperatur der neuen Aussenfläche (11) in der Zwischenzeit keinen Veränderungen unterzogen wird, die bedeutend zur Anwendung des Verfahrens sind.

**3.** Verfahren nach Patentanspruch 1 oder 2, **dadurch gekennzeichnet, dass** die Phase des Entfernens das Entfernen eines Endabschnittes (12) des Holzelementes enthält.

**4.** Verfahren nach einem jeden der vorstehenden Patentansprüche, **dadurch gekennzeichnet, dass** die Phase des Verwendens der Temperatur und der aktuellen Geschwindigkeit der Fortpflanzung oder einer anderen damit zusammenhängenden Grösse, um die Elastizität bei einer Referenztemperatur zu bestimmen, die Vorgehensphase der Kalkulation, basierend auf den genannten Messungen der aktuellen Geschwindigkeit der Fortpflanzung und der Temperatur des Holzelementes (1), einer Bezugsgeschwindigkeit der Fortpflanzung der Schallwelle in dem Holzelement (1) bei einer Referenztemperatur oder der anderen damit zusammenhängenden Grösse durch eine bekannte Formel enthält, und folglich die Vorgehenssphase der Bestimmung der Elastizität des Holzelementes (1) bei einer Referenztemperatur, basierend auf der Referenzgeschwindigkeit der Fortpflanzung.

**5.** Verfahren nach Patentanspruch 4, **dadurch gekennzeichnet, dass**, wenn die gemessene Temperatur höher ist als 0°C, die Kalkulationsphase der Geschwindigkeit der Fortpflanzung oder der anderen damit zusammenhängenden Grösse bei der Referenztemperatur erfolgt, indem angenommen wird, dass diese gleich der gemessenen ist.

**6.** Verfahren nach einem jeden der Patentansprüche von 1 bis 3, **dadurch gekennzeichnet, dass** die Phase des Verwendens der Temperatur und der aktuellen Geschwindigkeit der Fortpflanzung oder der anderen damit zusammenhängenden Grösse, um die Elastizität bei einer Referenztemperatur zu bestimmen, die Vorgehenssphase der Bestimmung der Elastizität bei der gemessenen Temperatur enthält, basierend auf den genannten Messungen der aktuellen Geschwindigkeit der Fortpflanzung oder der anderen damit zusammenhängenden Grösse, sowie die anschliessende Vorgehenssphase der Bestimmung der Elastizität des Holzelementes (1) bei einer Referenztemperatur, basierend auf der gemessenen Temperatur und der vorherigen Bestimmung, durchgeführt bei der gemessenen Temperatur.

7. Verfahren nach Patentanspruch 6, **dadurch gekennzeichnet, dass**, wenn die gemessene Temperatur höher ist als 0°C, die Phase der Bestimmung der Elastizität bei der Referenztemperatur erfolgt, indem angenommen wird, dass diese gleich der gemessenen Temperatur ist.

8. Verfahren nach einem jeden der vorstehenden Patentansprüche, **dadurch gekennzeichnet, dass** die Referenztemperatur entsprechend 20°C eingestellt ist.

9. Verfahren nach einem jeden der vorstehenden Patentansprüche, **dadurch gekennzeichnet, dass** die Kalkulation des Elastizitätsmodus basierend auf der Dichte des Holzelementes (1) durchgeführt wird, die vorher bestimmt oder geschätzt wurde.

10. Verfahren nach einem jeden der vorstehenden Patentansprüche, **dadurch gekennzeichnet, dass** es ebenfalls eine vorausgehende Kalibrierphase enthält.

11. Verfahren nach Patentanspruch 10, **dadurch gekennzeichnet, dass** die Kalibrierphase für die verschiedenen Holztypen und die verschiedenen Parameter des Holzelementes (1) das Messen der Geschwindigkeit der Fortpflanzung einer Schallwelle oder der anderen damit zusammenhängenden Grösse, und/oder die Bestimmung der Elastizität, für eine Anzahl von unterschiedlich möglichen Betriebstemperaturen enthält.


**Revendications**

1. Un procédé pour évaluer l'élasticité d'un élément en bois tel qu'un tronc ou une planche à une température de référence, comprenant les phases opérationnelles consistant à:

     générer une onde sonore dans l'élément en bois (1) ;
     déterminer la vitesse de propagation réelle de l'onde sonore dans l'élément en bois (1) ou une autre grandeur liée à celle-ci par une formule connue ;
     mesurer la température de l'élément en bois (1) ; et
     utiliser la température mesurée et la vitesse de propagation réelle mesurée, ou l'autre grandeur liée à celle-ci, pour évaluer l'élasticité à une température de référence prédéfinie ;
**caractérisé en ce que** la phase de mesure de la température est effectuée en mesurant la température interne de l'élément en bois (1),
et **en ce qu'**il comprend aussi, avant la phase de mesure de la température, une phase consistant à supprimer une partie de l'élément en bois (1) de manière à créer sur celui-ci une nouvelle face extérieure (11), la phase de mesure de la température étant effectuée en mesurant la température de surface au niveau de la nouvelle face extérieure (11).

2. Le procédé selon la revendication 1, **caractérisé en ce que** la phase de suppression et la phase de mesure sont effectuées en séquence dans un délai qui est tel que la température de surface de la nouvelle face extérieure (11) n'est pas soumise, pendant ce temps-là, à des variations significatives pour la mise en oeuvre dudit procédé.

3. Le procédé selon la revendication 1 ou 2, **caractérisé en ce que** la phase de suppression comprend la suppression d'une portion d'extrémité (12) de l'élément en bois.

4. Le procédé selon l'une quelconque des revendications précédentes, **caractérisé en ce que** la phase d'utilisation de la température et de la vitesse de propagation réelle ou de l'autre grandeur liée à celle-ci, pour évaluer l'élasticité à la température de référence, comprend la phase opérationnelle consistant à calculer, sur la base desdites mesures de la vitesse de propagation réelle et de la température de l'élément en bois (1), une vitesse de propagation de référence de l'onde sonore dans l'élément en bois (1) à la température de référence, ou une autre grandeur liée à celle-ci par une formule connue, puis la phase opérationnelle consistant à évaluer l'élasticité de l'élément en bois (1) à la température de référence, sur la base de ladite vitesse de propagation de référence.

5. Le procédé selon la revendication 4, **caractérisé en ce que**, lorsque la température mesurée est supérieure à 0°C, la phase de calcul de la vitesse de propagation, ou de l'autre grandeur liée à celle-ci, à la température de référence est effectuée en supposant qu'elle est égale à celle mesurée.

6. Le procédé selon l'une quelconque des revendications de 1 à 3, **caractérisé en ce que** la phase d'utilisation de la température et de la vitesse de propagation réelle, ou de l'autre grandeur liée à celle-ci, pour évaluer l'élasticité à la température de référence, comprend la phase opérationnelle consistant à évaluer l'élasticité à la température mesurée sur la base desdites mesures de la vitesse de propagation réelle ou de l'autre grandeur liée à celle-ci, et la phase opérationnelle suivante consistant à évaluer l'élasticité de l'élément en bois (1) à la température de référence sur la base de la température mesurée et de l'évaluation précédente effectuée à la température mesurée.

7. Le procédé selon la revendication 6, **caractérisé en ce que**, lorsque la température mesurée est supérieure à 0°C, la phase d'évaluation de l'élasticité à la température de référence est effectuée en supposant qu'elle est égale à celle à la température mesurée.

8. Le procédé selon l'une quelconque des revendications précédentes, **caractérisé en ce que** la température de référence est définie comme étant égale à 20°C.

9. Le procédé selon l'une quelconque des revendications précédentes, **caractérisé en ce qu'**un calcul du module d'élasticité est effectué sur la base de la densité de l'élément en bois (1) précédemment déterminée ou estimée.

10. Le procédé selon l'une quelconque des revendications précédentes, **caractérisé en ce qu'**il comprend aussi une phase de réglage préliminaire.

11. Le procédé selon la revendication 10, **caractérisé en ce que** la phase de réglage consiste à mesurer, pour différents types de bois et différents paramètres de l'élément en bois (1), la vitesse de propagation d'une onde sonore ou l'autre grandeur liée à celle-ci, et/ou à évaluer l'élasticité, pour une pluralité de différentes températures opérationnelles possibles.

FIG. 1

FIG. 2

FIG. 3

FIG. 4

FIG. 5

EP 2 369 340 B1

FIG. 7

FIG. 6

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 4435975
- US 6026089 A
- US 20080197054 A
- US 20100064810 A

**Non-patent literature cited in the description**

- **HELMUT BÄCHLE et al.** The influence of temperature on the velocity of sound in green pine wood. *Holz als Roh- und Werkstoff,* 2006, vol. 64, 429-430
- **SANDOZ.** Moisture content and temperature effect on ultrasound timber grading. *Wood Sci. Technol,* 1993, vol. 27, 373-380
- **JULIAN MORENO CHAN.** *Moisture content in radiata pine wood: Implications for wood quality and water-stress response,* 2007